# EUROPEAN PATENT APPLICATION

(11) **EP 4 101 444 A1**
(43) Date of publication of application: **14.12.2022**
(21) Application number: 20917601.5
(22) Date of filing: 14.12.2020
(51) Int. Cl.: A61K 31/045, A61K 35/64

(54) **ORAL SUSPENSION WITH ANTI-ULCEROUS AND CHEMOPROTECTIVE EFFECT ON COLON CANCER AND PREPARATION METHOD THEREOF**

(30) Priority: 03.02.2020 CU 20200007
(71) Applicant: Centro Nacional De Investigaciones Cientificas (CNIC), La Habana 113000 (CU); Empresa Laboratorio Farmaceutico Liquidos Orales Medilip, Granma 85100 (CU)
(72) Inventor: GONZÁLEZ CANAVACIOLO, Víctor Luis, La Habana 11300 (CU); VICENTE MURILLO, Roxana, La Habana 10700 (CU); RODRÍGUEZ ZAMORA, Reynerio, Granma 85100 (CU); BENITEZ GUERRA, Niurka, Granma 85100 (CU); RODRIGUEZ LEYES, Eduardo Antonio, La Habana 17100 (CU); MOLINA CUEVAS, Vivian, La Habana 11300 (CU); MENDOZA CASTAÑO, Sarahí, La Habana 11300 (CU); OYARZÁBAL YERA, Ámbar, La Habana 17100 (CU)
(74) Representative: Herrero & Asociados, S.L.
(86) International application number: PCT/CU2020/050008
(87) International publication number: WO 2021/155871

(57) **Abstract**

A new pharmaceutical composition consisting of an oral aqueous suspension for the prevention and treatment of gastric ulcers and colon cancer is disclosed, as well as the procedure for preparing it. This suspension is characterized by containing a purified extract with fatty alcohols from beeswax, microcrystalline cellulose and excipients accepted by the pharmaceutical industry. The method of preparing this suspension is characterized in that the fatty alcohol extract is subjected to a process of particle size reduction to <1.5 micrometers in the presence of an emulsifying agent at >1000 rpm; the mixture of said active ingredient with microcrystalline cellulose in the proportion and concentration in which they are formulated, makes these components act synergistically, conferring powerful antiulcer and chemoprotective effects to this composition.

## Description

This invention is related to a new pharmaceutical composition, consisting of an oral aqueous suspension, as well as its production process. This suspension has antiulcer effects on the gastric mucosa and chemoprotective effects against colon cancer, and is characterized by containing, as active ingredients, a purified extract with fatty alcohols from the refined wax of Apis mellifera bees, which particle size has been reduced by a technological procedure to <1.5 micrometers, and microcrystalline cellulose. The purified extract of beeswax, in addition to fatty alcohols, contains diols, paraffins and potassium salts of fatty acids as minor components. This new composition also presents a series of excipients accepted by the pharmaceutical industry, including: one or more emulsifying agents, used in the step of reducing the particle size of the fatty alcohol extract, as well as wetting agents, sweeteners, suspending agents, preservatives, flavorings and water.

Gastric ulcer is an injury that extends to the mucosal layer and sometimes to the muscular layer of the stomach, forming a cavity with acute and chronic inflammation around it, this being the main cause of digestive bleeding, one of its main complications (Valley 2002, Sadic 2009). Other complications include perforation, penetration, and obstruction, all of which lead to significant impairment of the patient's quality of life (Sung 2010). The ulcer is produced by an imbalance between aggressive (acid secretion, pepsin, H. pylori, nonsteroidal anti-inflammatory drugs) and defensive factors (mucus and bicarbonate secretion, blood microcirculation, prostaglandins, growth factors), being H. pylori infection and the continued use of non-steroidal anti-inflammatory the main causes (Ramakrishnan 2007). Despite current advances in H. pylori eradication therapy, the mortality rate from peptic ulcer complications remains stable. In clinical practice, the most commonly used drugs for the treatment of gastric ulcers are proton pump inhibitors and H2 receptor antagonists, although these have various adverse effects such as nausea, constipation, gynecomastia and impotence (Forgerini 2018). These effects limit their use and justify the search and use of new pharmaceutical compositions, effective as antiulcer agents, and with higher safety profiles. The most widely used pharmacological models for the study of potentially anti-ulcer substances are based on inducing ulcers with alkaline substances, ethanol, or by ligation of the pylorus, as a way of covering different possible mechanisms by which the substance to be evaluated exerts its anti-ulcer effect.

On the other hand, cancer is one of the current leading causes of death, with more than 8 million deaths annually worldwide, with colon cancer being one of the five leading causes of death worldwide with an increasing trend, especially in countries with fewer resources (Torre 2015, Chatenoud 2014). Taking into account the high incidence of this disease, as well as the fact that its treatment is generally surgical, followed by chemotherapy, the search for and use of chemoprotective pharmaceutical compositions that can be used in the prevention and treatment of patients with this type of cancer or with risk factors for it, such as ulcerative processes in the digestive tract (ulcerative colitis, Crohn's disease and others) is justified. The most widely used pharmacological models for the study of potentially chemoprotective substances against this disease are based on the use of cancer-inducing agents. Among the latter, one of the most effective and used is 1,2-dimethylhydrazine. The metabolism of this carcinogen begins to generate cytotoxic substances for colonocytes within a few hours of induction (Corpet 2003, Reddy 2004).

Bearing in mind that both gastric ulcer and colon cancer affect the quality of life and can cause the death of those who suffer from it, constituting important health problems worldwide, as well as the fact that existing conventional therapies are associated with various adverse effects that limit their clinical use and do not always manage to cure or prevent the incidence of these diseases, it is necessary to search for effective therapeutic agents for both medical conditions. The need for new safe and effective anti-ulcer and anti-cancer therapeutic strategies is a current problem.

The purified extract of refined beeswax used in the composition object of the present invention, as well as its production process, were previously patented, said extract having been claimed as a natural mixture of higher primary aliphatic alcohols for the treatment of gastric and duodenal ulcers, with anti-inflammatory activity, whose most suitable form of administration was in the form of tablets, pills or capsules (CU 22412 A1), that is, in finished solid forms. In accordance with the above, a tablet with this active ingredient, standardized to a dose of 50 mg of fatty alcohols, was registered as a nutritional supplement in Cuba and in other countries as a functional food or alternative medicine. However, this formulation has several drawbacks such as: a large size (655 mg) that leads to a high mass of excipients and high production costs; growing rejection in different countries of the use of two of its excipients (croscarmellose sodium and polyvinylpyrrolidone); and very low bioavailability of the fatty alcohols it contains (<10%).

It should be noted that the pharmacological potency, as an antiulcer agent, of said tablet is much lower than the potency reached by the suspension object of the present invention. When the fatty alcohol extract is subjected to technological processes of shearing or precipitation by temperature change with agitation, in the presence of specific emulsifying agents, to reduce its particle size, and subsequently being formulated in an aqueous suspension, mixed with microcrystalline cellulose in proportions and specific concentrations, its pharmacological efficacy is synergistically enhanced, which was not deductible from the state of the art. The aqueous suspension object of this invention significantly exceeds the antiulcer effect of both fatty alcohol extract in tablet form and microcrystalline cellulose, and has a powerful chemoprotective effect on colon cancer that had not been previously reported for neither of these two substances, which can be seen in the embodiment examples.

On the other hand, microcrystalline cellulose (derived from alpha cellulose) has been used mainly in the manufacture of creams and solid suspensions for cosmetics, detergents and as a stabilizer in food (Nsor-Atindana, 2017). In the pharmaceutical industry it is used for the preparation of tablets and capsules, as a compression and filling agent, although its bile acid sequestering capacity has also been reported (Paniagua 1999) and it has been used as an active ingredient in a suspension with an antiulcer effect, at a 12% dose (Barzaga 2004). It should be noted that the pharmacological effect reported for said 12% suspension is much lower than that achieved with the suspension disclosed in the present invention, whose dose of microcrystalline cellulose is much lower (≤ 2%), as can be seen in the embodiment example number 3. This is the result of the synergistic effect that occurs when microcrystalline cellulose is mixed in specific proportions and concentrations with the purified extract of fatty alcohols from beeswax, which enhances its antiulcer effect and makes the composition acquire a powerful chemoprotective effect against colon cancer not found for the previous formulations, which was not deductible from the state of the art.

The use of refined beeswax and microcrystalline cellulose in the pharmaceutical industry has so far been limited to the formulation of solid medications whose formulations allow a controlled release of the active ingredient (Kállai 2010; Reiner, 2018), or the use of both as inert filler excipients in suppositories or other formulations (Lee, 2018). The joint use of an extract of fatty alcohols from beeswax and microcrystalline cellulose in an aqueous suspension, where both substances act synergistically, providing powerful antiulcer and chemoprotective effects to the composition, has also not been reported prior to the present invention. When using both substances in the form of tablets (CU 22412 A1) the effects observed with the aqueous suspension described in the present invention are not achieved either, as can be seen in the Embodiment Examples.

The procedure for obtaining the pharmaceutical composition object of the present invention is characterized by: A) mixing an extract of fatty alcohols extracted from beeswax (1-2%), with polyoxyethylene hydrogenated castor oil or polysorbate (2- 4%), substances used as emulsifying agents; B) subjecting said mixture to a technological process to reduce the particle size of the extract to <1.5 micrometers, which can be done with homogenizing equipment capable of shearing the particles at >1000 rpm and >80°C for >1 minute , or subjecting said mixture to a stirring speed > 1000 rpm and > 80°C for > 1 minute and then lowering the temperature to < 60°C with the same stirring speed; C) keeping the agitation, incorporate microcrystalline cellulose as the second active ingredient (1-2%), methyl and propyl parabens as preservative agents (0.1-0.6%), glycerin as wetting agent (20-30 %), sorbitol or sodium saccharin as sweetening agents (1-6%), carboxymethylcellulose as suspending agent (0.1-1%), essential oils as flavoring agents (0.01-0.05%) and purified water (50 -70%). The result of this procedure is a new pharmaceutical composition, characterized by being an aqueous suspension for oral use, whose active ingredients: fatty alcohol extract and microcrystalline cellulose act synergistically, giving it powerful antiulcer and chemoprotective effects.

This new pharmaceutical composition is intended to prevent or treat ulcerative processes and other related conditions, given its protective nature on the digestive tract, and its chemoprotective effects on colon cancer. The proportion in which the active ingredients are present in this pharmaceutical composition is the result of several studies carried out with a view to standardizing the contents of both active ingredients and excipients in the final composition at specific concentrations and proportions that guarantee the desired pharmacological effects, which are the result of a synergistic action of the active ingredients it contains. The powerful pharmacological effects of the present composition are much higher than the effects of separately administering both active ingredients, even at doses higher than the doses in which they are present in the present composition, which can be seen in the Embodiment Examples and demonstrates the occurrence of a synergistic effect not deductible from the state of the art.

The advantage of this composition over other existing ones is that its antiulcer efficacy is superior to that of currently registered tablets with 50 mg of beeswax fatty alcohols, and also superior to that of the 12% microcrystalline cellulose suspension, whose development was previously reported; in other words, the present composition has antiulcer effects that are much higher than those of both previous formulations, even with lower doses of the active ingredients. On the other hand, it has been shown that the present composition has an effective chemoprotective effect on colon cancer, significantly higher than that of both active ingredients separately in similar suspensions, as can be seen in Embodiment Example number 6. This effect had not been observed before for the purified extract of beeswax or for microcrystalline cellulose, being the result of a synergistic action of both substances in the suspension object of the present invention. Additionally, the industrial production of this suspension is cheaper than the production of tablets with 50 mg of fatty alcohols and does not contain any excipients with limitations of use in the current pharmaceutical industry.

This invention has an industrial application, the composition is novel, and both the suspension itself and its production process are inventive, since it is not deductible from the state of the art that the mixture of two different antiulcer active ingredients, in specific proportions and concentrations, neither of which has demonstrated a chemoprotective effect on carcinogenic processes, shows a synergistic effect with pharmacological benefits on gastric ulcers and a chemoprotective effect on colon cancer. The present invention is related to the food and pharmaceutical industries, since the composition obtained can be used as a nutritional supplement, due to its beneficial preventive effects on the digestive tract, and also as a medicine for the treatment of ulcers and cancer.

### Example 1.

In an auxiliary stainless-steel container with a 50 L capacity (R1), 12 L of purified water were added and 1 kg of microcrystalline cellulose was incorporated little by little, at intervals and with stirring. It was stirred at 60 rpm for 30 minutes, until a homogeneous paste was obtained. 5 kg of 70% sorbitol and 24 kg of glycerin were added with stirring and stirring was continued for 10 minutes to obtain Mixture 1 (M1). On the other hand, in a stainless-steel reactor with a 300 L capacity, 60 L of water were heated to 90ºC, 10 liters were separated in an auxiliary container (R2), 3 kg of polysorbate 80 were incorporated with stirring at 1200 rpm and it was stirred for 5 minutes at 1200 rpm. 1.2 kg of the purified extract of fatty alcohols was added little by little and at intervals and stirring was maintained for 10 minutes to obtain Mixture 2 (M2), after which the temperature was lowered to 30 ± 2 ºC, maintaining the agitation. In another vessel, 0.18 kg of methylparaben and 0.02 kg of propylparaben were dissolved in 1.5 liters of ethanol with stirring at 60 rpm. 0.2 kg of sodium carboxymethylcellulose A/V were moistened, 0.03 kg of flavoring were incorporated and stirred for 5 minutes to obtain Mixture 3 (M3).

In another 5 L auxiliary vessel, 1 kg of glycerin was mixed with 0.5 L of ethanol and stirred for 5 minutes at 60 rpm to obtain Mixture 4 (M4). In the reactor where the mixture M2 was found, M1 was incorporated, dragging the remains with the minimum amount of water separated in R2 and stirred for 10 minutes at 60 rpm. After this time, M3 was incorporated and the remains were dragged with M4. Subsequently, what remained of the suspension was dragged with the hot water that was in R2 and stirred for 10 minutes. The stirring was then stopped and the mixture was allowed to stand for 10 hours. After this time, the remaining alcohol was sprinkled on the surface of the suspension and made up to the mark with water to complete 100 liters of suspension. It was stirred for 5 minutes at 1200 rpm, the suspension was filtered and a sample was taken for quality control. The final preparation was packed in amber glass bottles that were covered with plastic screw caps and the bottles were stored at room temperature.

### Example 2.

In an auxiliary stainless-steel container with a 50 L capacity (R1), 12 L of purified water were added and 1 kg of microcrystalline cellulose was incorporated little by little, at intervals and with stirring at 40 rpm. It was stirred for 30 minutes, until a homogeneous paste was obtained. 5 kg of 70% sorbitol and 24 kg of glycerin were added with stirring and stirred at 40 rpm for 10 minutes to obtain Mixture 1 (M1). On the other hand, in a stainless-steel reactor with a 300 L capacity, equipped with a Polytron-type homogenizer system, 60 L of water were heated to 90ºC, 10 liters were separated in an auxiliary container (R2), and incorporated with stirring. 9000 rpm 3 kg of cremophor RH 40 and stirred for 5 minutes. 1.2 kg of the purified extract of fatty alcohols was added little by little and at intervals and stirring was maintained for 10 minutes to obtain Mixture 2 (M2), after which the temperature was lowered to 30 ± 2 ºC, maintaining the agitation. In another container, 0.18 kg of methylparaben and 0.02 kg of propylparaben were dissolved in 1.5 liters of ethanol with stirring at 40 rpm. 0.3 kg of sodium carboxymethylcellulose A/V were moistened, 0.03 kg of flavoring were incorporated and stirred for 5 minutes to obtain Mixture 3 (M3).

In another 5 L auxiliary vessel, 1 kg of glycerin was mixed with 0.5 L of ethanol and stirred for 5 minutes to obtain Mixture 4 (M4). In the reactor where the mixture M2 was found, M1 was incorporated, dragging the remains with the minimum amount of water separated in R2 and stirred for 10 minutes at 40 rpm. After this time, M3 was incorporated and the remains were dragged with M4. Subsequently, what remained of the suspension was dragged with the hot water that was in R2 and stirred for 10 minutes at 1000 rpm. The stirring was then stopped and the mixture was allowed to stand for 12 hours. After this time, the remaining alcohol was sprinkled on the surface of the suspension and made up to the mark with water to complete 100 liters of suspension. It was stirred for 5 minutes at 1000 rpm, the suspension was filtered and a sample was taken for quality control. The final preparation was packed in amber glass bottles that were covered with plastic screw caps and the bottles were stored at room temperature.

### Example 3.

The composition obtained in example 1 (FAMC) was subjected to a preclinical test with animals, in which its antiulcer effect was compared with the antiulcer effects of the registered fatty alcohol tablet (RAT), and suspensions of Fatty Alcohols (FA) and microcrystalline cellulose (MC), respectively, the latter two prepared with doses of FA and MC equal to the doses of both substances in the FAMC suspension. The experimental model used in this trial was the induction of gastric ulcers in rats by sodium hydroxide.

Male Sprague-Dawley rats (250-300 g) were used, which were adapted for 7 days to the usual laboratory conditions at 20-25⁰C, relative humidity of 60 ± 5%, light/dark cycles of 12 hours and with free access to water and standard feed for rodents. After completing the quarantine, the animals were randomly distributed into 10 experimental groups (10 rats/group): a negative control that only received the vehicle and 9 groups in which the ulcer was induced with 0.2 N sodium hydroxide, among these a positive control not treated with any antiulcer agent and 8 groups treated with the substances to be evaluated as shown in Table 1. The suspensions were administered as they are, while the RAT was prepared in acacia gum/water vehicle (1%). The use of a negative control group (not subjected to damage with sodium hydroxide), as well as that of a positive control group (subjected to damage and without treatment with any substance) allowed corroborating the validity of the model under the experimental conditions, and comparing the anti-ulcer efficacy of the substances evaluated.

All treatments and sodium hydroxide were administered orally, as a single dose, by intragastric intubation. The selected doses are within the range of effective doses proven in previous preliminary experiences. To induce gastric ulcer, the animals were fasted for 24 hours prior to the experiment, with free access to water. One hour after the administration of single doses of the vehicle, and the different treatments, each rat received the ulcer-inducing agent (1 mL/200 g) by gastric intubation. One hour later, the rats were sacrificed in a halothane atmosphere, the stomachs were removed and opened at the greater curvature.

Gastric mucosal lesions were quantified by two independent blinded observers. Lesion score was defined as the sum total of lesion sizes in mm² (Ohara 1992). Statistical comparison between the mean ulcer areas in the different groups was performed using the non-parametric Mann Whitney U test. A priori, a significance level of α = 0.05 was set and the commercial package Statistic for Windows (Release 4.2, StatSoft, Inc USA) was used.

**Table 1. Effects of FAMC, MC, FA suspensions, and RAT on sodium hydroxide-induced gastric ulcer in rats.**

| **Treatment** | **Doses (mg/kg/day)** | **Ulcer index (mm2)** | **I (%)** |
|---|---|---|---|
| Negative control (Vehicle) | 0 | 0 ± 0.00*** | - |
| Positive Control (Vehicle + NaOH) | 0 | 83.70 ± 13.8 | -- |
| MC + NaOH | 25 | 80 ± 10.6 | 4.4 |
| MC + NaOH | 200 | 75.4 ± 12.5 | 9.9 |
| FA + NaOH | 25 | 67.2 ± 11.2 | 19.7 |
| FA + NaOH | 200 | 50.3 ± 13.5** | 39.9 |
| RAT + NaOH | 25 | 51.4 ± 10.4 ** Δ | 38.6 |
| RAT + NaOH | 200 | 40.5 ± 12.70*** Δ | 51.6 |
| FAMC + NaOH | 25 | 11.3 ± 3.00*** °a b | 86.5 |
| FAMC + NaOH | 200 | 1.2 ± 0.3*** ° a b | 98.6 |

| | | | |
|---|---|---|---|
| FAMC: Suspension of fatty alcohols plus microcrystalline cellulose MC: Microcrystalline cellulose suspension FA: Suspension of Fatty Alcohols RAT: Fatty alcohol tablet registered as a nutritional supplement I inhibition, Data as Mean ± SEM (standard error of the mean) * p <0.05, ** p <0.01, *** p <0.0001, comparison vs positive control Δ p< 0.05, ° p <0.0001 comparison vs similar dose of FA, a p <0.0001 comparison vs similar doses of MC b p <0.0001 comparison vs similar doses of RAT | | | |

As shown in Table 1, the oral administration of NaOH (0.2 N) produced gastric lesions in the animals of the positive control group compared to the healthy animals (negative control), which demonstrates the validity of the model in the experimental conditions

Oral administration with FAMC, FA, and RAT reduced the gastric ulcer index, while CM only produced slight reductions that were not significant. The FAMC suspension (25 and 200 mg/kg) produced a reduction in the gastric ulcer index, which was significant not only compared to the positive control group, but also compared to the same doses of the MC, FA and RAT suspensions.

Therefore, the highest anti-ulcer efficacy was achieved with the FAMC suspension, especially since it produced ~100% (total) inhibition of the gastric ulcer index with the highest dose tested of 200 mg/kg.

In addition, the FAMC suspension produced higher inhibition percentages than the sum of those achieved with the MC and FA suspensions separately, showing a synergism in the FAMC, where the presence of the FA extract and the MC in the proportions and concentrations in which appear promotes greater anti-ulcer efficacy.

It should also be noted that the antiulcer effect of the FAMC suspension is much higher than the effect reported for the 12% CM suspension (Barzaga 2004), both evaluated in alkaline gastritis ulcer induction models. Thus, the percentages of inhibition achieved with 25 and 200 mg/kg of the FAMC suspension (86.5 and 98.6%, respectively) are higher than that reported for MC, which was 50% at a higher dose (356.38 mg/kg) (Barzaga 2004), which is in correspondence with the synergistic effect of the FAMC observed in this example.

It is also worth noting that, in the present example, the lowest dose tested of 25 mg/kg of the FAMC suspension produced a high percentage of inhibition of 86.5% on gastric ulcers, which indicates that in addition to a high efficacy, the FAMC suspension also has high power.

### Example 4.

The composition obtained in example 2 (FAMC) was subjected to a preclinical test with animals, in which its antiulcer effect was compared with the antiulcer effects of the registered fatty alcohol tablet (RAT), and suspensions of Fatty Alcohols (FA) and microcrystalline cellulose (MC), the latter two prepared with doses of FA and MC equal to the doses of both substances in the FAMC suspension. The experimental model used in this trial was the induction of gastric ulcers in rats by ethanol.

Male Sprague-Dawley rats (250-300 g) were used, which were adapted for 7 days to the usual laboratory conditions at 20-25 °C, relative humidity of 60 ± 5%, light/dark cycles of 12 hours and with free access to water and standard feed for rodents. After completing the quarantine, the animals were randomly distributed into 11 experimental groups (10 rats/group): a negative control that only received the vehicle and 10 groups in which the ulcer was induced with 60% ethanol, among these a control positive not treated with any antiulcer agent, 8 groups treated with the substances to be evaluated and a group treated with Omeprazole as a reference substance, as shown in Table 2. The suspensions were administered as they are, while a tablet was prepared in a suspension with 1% acacia gum.

The use of a negative control group (not subjected to damage with ethanol), as well as a positive control group (subjected to damage and without treatment with any substance) made it possible to verify the functioning of the model and compare the anti-ulcer efficacy of the substances evaluated. All treatments and ethanol were administered orally, as a single dose, by intragastric intubation. The selected doses are within the range of effective doses verified in previous preliminary experiences and the dose of omeprazole (20 mg/kg) has also been effective in this model. To induce gastric ulcer, the animals were fasted for 24 hours prior to the experiment, with free access to water. One hour after the administration of single doses of the vehicle, and the different treatments, each rat received the ulcer-inducing agent (1 mL/200 g) by gastric intubation. One hour later the rats were sacrificed in a halothane atmosphere, the stomachs were removed and opened at the greater curvature. The quantification of the gastric ulcer index was performed as described in example 3.

The results on the ethanol-induced ulcer index (table 2) show that the oral administration of ethanol produced the formation of gastric lesions in the animals of the positive control group compared to the animals of the negative control group (healthy). The animals treated with the FAMC suspension at doses of 25 and 200 mg/kg were the ones that showed a lower ulcer index compared to the animals with damage (positive control). Treatment with said suspension markedly and significantly decreased the gastric ulcer index, reaching 93.95 and 99.85% inhibition at doses of 25 and 200 mg/kg, respectively. Statistical comparisons between the effects of the different treatments used, at similar doses, showed significant differences, highlighting that the FAMC suspension showed the best results, even higher than the sum of the percentages of inhibition of the FA and MC suspensions, demonstrating a greater protection of the gastric mucosa. The reference substance used (Omeprazole) also decreased the gastric ulcer index, which corroborates the validity of the results obtained in our experimental conditions. It is noteworthy that the FAMC suspension achieved superior effects to those of Omeprazole, although they are not administered at the same doses, since it was not the objective of the study to compare both substances.

**Table 2. Effects of FAMC, MC, FA suspensions, and RAT on ethanol-induced gastric ulcer in rats.**

| **Treatment** | **Doses (mg/kg/day)** | **Ulcer Index (mm²)** | **I (%)** |
|---|---|---|---|
| Negative Control (Vehicle) | 0 | 0.15 ± 0.07*** | - |
| Positive Control (Vehicle) | 0 | 169.09 ± 12.8 | -- |
| MC+ ethanol | 25 | 155.5 ± 11.6 | 8 |
| MC+ ethanol | 200 | 133.3 ± 13.7 | 21.1 |
| FA + ethanol | 25 | 149.13 ± 12.2 | 11.7 |
| FA + ethanol | 200 | 89.19 ± 14.5** | 47.3 |
| RAT + ethanol | 25 | 106.60 ± 11.40** Δ | 37 |
| RAT + ethanol | 200 | 41.74 ± 13.70*** Δ | 75.4 |
| OMP + ethanol | 20 | 35.97 ± 14.48*** | 78.8 |
| FAMC + ethanol | 25 | 10.38 ± 3.00*** °a b | 93.95 |
| FAMC + ethanol | 200 | 0.40 ± 0.34*** ° a b | 99.85 |

| | | | |
|---|---|---|---|
| FAMC: Suspension of fatty alcohols plus microcrystalline cellulose MC: Microcrystalline cellulose suspension FA: Suspension of Fatty Alcohols RAT: Fatty alcohol tablet registered as a nutritional supplement I inhibition, Data as Mean ± SEM (standard error of the mean) * p <0.05, ** p <0.01, *** p <0.0001, comparison vs positive control Δ p< 0.05, ° p <0.0001 comparison vs similar dose of FA, a p <0.0001 comparison vs similar doses of MC b p <0.0001 comparison vs similar doses of RAT (Mann Whitney U test) | | | |

As a conclusion of the test presented in this example, it can be stated that oral administration with the FAMC suspension markedly and significantly inhibited the formation of gastric ulcer induced by ethanol in rats, with a higher efficacy than that achieved with the registered tablet and with the suspensions FA and MC at the same doses. Additionally, it was observed that the efficacy of the FAMC suspension was greater than the sum of the efficacy of the FA and MC suspensions in both doses evaluated, which demonstrates the existence of a synergistic effect in the composition object of the present invention.

### Example 5.

The composition obtained in example 2 (FAMC) was subjected to a preclinical test with animals, in which its antiulcer effect was compared with the antiulcer effects of the registered fatty alcohol tablet (RAT), and suspensions of Fatty Alcohols (FA) and microcrystalline cellulose (MC), the latter two prepared with doses of FA and MC equal to the doses of both substances in the FAMC suspension. The experimental model used in this test was that of the induction of gastric ulcers in rats by ligation of the pylorus, which is an acid-dependent model, unlike the model presented in the previous example.

Male Sprague-Dawley rats (250-300 g) were used, which were adapted for 7 days to the usual laboratory conditions at 20-25 °C, relative humidity of 60 ± 5%, light/dark cycles of 12 hours and with free access to water and standard feed for rodents. After completing the quarantine, the animals were randomly distributed into 11 experimental groups (10 rats/group): a negative control that only received the vehicle and 10 groups in which the ulcer was induced by ligation of the pylorus, among these a positive control not treated with any antiulcer agent, 8 groups treated with the substances to be evaluated and a group treated with Omeprazole as a reference substance, as shown in Table 3. The suspensions were administered as they are, while a suspension with 1% acacia gum was prepared to administer the tablet. The use of a negative control group (not subjected to pylorus ligation), as well as that of a positive control (subjected to damage and without treatment with any substance) made it possible to verify the functioning of the model and compare the anti-ulcer efficacy of the substances evaluated.

All treatments were administered orally, as a single dose, by intragastric intubation. The selected doses are within the range of effective doses verified in previous preliminary experiences and the dose of omeprazole (20 mg/kg) has also been effective in this model. To induce gastric ulcer, the animals were fasted for 24 hours prior to the experiment, with free access to water. One hour after the administration of single doses of the vehicle, and the different treatments, the animals were anesthetized by intraperitoneal injection with sodium thiopental (35 mg/kg), a 2 cm-long incision was made in the anterior third of the line abdominal media, locating the stomach and ligating the pyloric sphincter with silk suture number 2 and the abdominal wall was sutured with silk thread. One hour later, the rats were sacrificed in a halothane atmosphere, the stomachs were removed and the gastric juice collected in test tubes. The latter was centrifuged at 3000 rpm, the supernatant separated and quantified in ml. The stomachs were opened through the greater curvature and the gastric ulcer index was quantified as described in example 3.

The results on the index of ulcer induced by pylorus ligation (Table 3) show that the formation of gastric lesions occurred in the animals of the positive control group with respect to the animals of the negative control group (healthy). The animals treated with the FAMC suspension at doses of 25 and 200 mg/kg were the ones that showed a lower ulcer index compared to the animals with damage (positive control). Treatment with said suspension markedly and significantly decreased the gastric ulcer index, reaching 76.3 and 99.7% inhibition at doses of 25 and 200 mg/kg, respectively. Statistical comparisons between the effects of the different treatments at similar doses showed that the FAMC suspension is the substance evaluated that presents the greatest anti-ulcer efficacy, since it was significantly superior to RAT, and to the suspensions with the respective monotherapies of FA and MC, respectively. In addition, the fact that the inhibition achieved by the FAMC suspension was greater than the sum of those achieved individually with MC and FA, demonstrates the synergistic effect of the presence of these two components in the mixture of the FAMC suspension.

Meanwhile, Omeprazole, the reference substance, significantly decreased the gastric ulcer index, which corroborates the validity of the results obtained in our experimental conditions. The volume of gastric juice was not modified by the suspensions of FAMC, MC, FA, or by RAT, which suggests that the antiulcer action of these substances does not depend on an effect on the formation of gastric acid. Only omeprazole reduced this variable, which is consistent with its mechanism of action as an antisecretory proton pump inhibitor.

**Table 3. Effects of FAMC, MC, FA suspensions, and RAT on gastric ulcer induced by pylorus ligation in rats.**

| **Treatment** | **Doses (mg/kg/day)** | **Ulcer Index (mm²)** | **I (%)** | **Gastric juice volume** |
|---|---|---|---|---|
| **Negative Control (Vehicle)** | 0 | 0,0 ± 0,00*** | - | 0,0 ± 0,00*** |
| **Positive Control (Vehicle + pylorus ligation)** | 0 | 23,2 ± 3,2 | -- | 9,9 ± 1,2 |
| **MC+ pylorus ligation** | 25 | 21,1 ± 1,6 | 9 | 10 ± 1,05 |
| **MC+ pylorus ligation** | 200 | 20,5 ± 1,7 | 11,6 | 9,5 ± 1,2 |
| **FA + pylorus ligation** | 25 | 18,3 ± 2,2 | 21,1 | 9,8 ± 1,3 |
| **FA + pylorus ligation** | 200 | 13,3 ± 3,1** | 42,7 | 9,7 ± 1,1 |
| **RAT + pylorus ligation** | 25 | 16,1 ± 2,40** | 30,6 | 9,8 ± 1,1 |
| **RAT + pylorus ligation** | 200 | 10,6 ± 3,2 *** | 54,3 | 9,6 ± 0,8 |
| **OMP** + **pylorus ligation** | 20 | 3,9 ± 0,5 *** | 83,2 | 3,4 ± 0,4 *** |
| **FAMC** + **pylorus ligation** | 25 | 5,5 ± 0,7 *** °a b | 76,3 | 9,5 ± 0,7 |
| **FAMC** + **pylorus ligation** | 200 | 0,3 ± 0,3*** ° a b | 98,7 | 9,4 ± 0,9 |

| | | | | |
|---|---|---|---|---|
| FAMC: Suspension of fatty alcohols plus microcrystalline cellulose MC: Microcrystalline cellulose suspension FA: Suspension of Fatty Alcohols RAT: Fatty alcohol tablet registered as a nutritional supplement I inhibition, Data as Mean ± SEM (standard error of the mean) * p <0.05, ** p <0.01, *** p <0.0001, comparison vs positive control ° p <0.0001 comparison vs similar dose of FA, a p <0.0001 comparison vs similar doses of MC b p <0.0001 comparison vs similar doses of RAT (Mann Whitney U test) | | | | |

As a conclusion of the test presented in this example, it can be stated that oral administration with the FAMC suspension markedly and significantly inhibited the formation of gastric ulcer induced by ligation of the pylorus in rats, with a higher efficacy than that achieved with the registered tablet and with FA and MC suspensions at the same doses. Additionally, it was observed that the efficacy of the FAMC suspension was greater than the sum of the efficacy of the FA and MC suspensions in both doses evaluated, which demonstrates the existence of a synergistic effect in the composition object of the present invention.

### Example 6.

The composition obtained in example 1 (FAMC) was subjected to a preclinical test with animals, in which its chemoprotective effect was compared with the chemoprotective effects of the registered fatty alcohol tablet (RAT), and suspensions of Fatty Alcohols (FA) and microcrystalline cellulose (MC) at equal concentrations to those present in the FAMC composition. For the evaluation, colon cancer was induced to Holtzmann rats with 1,2-dimethylhydrazine (DMH). The animals, with three months of age and 250 ± 20 g; were randomly distributed into the following groups: sodium polysorbate at 2 mL/kg; DMH 20mg/kg; and DMH plus FAMC, RAT, FA; and MC suspensions.

DMH was prepared at 4 mg/mL in distilled water, with 0.4 mg/mL EDTA as stabilizer at pH 6.5. It was administered subcutaneously at 20 mg/kg body weight once a week for 18 weeks, while the suspensions and polysorbate were administered orally for the 18-week study period. After the scheduled time, one hour after the last administration, the rats were sacrificed with 100 mg/kg pentobarbital, and the colon was removed for histopathological analysis, it was gently washed with saline solution to remove blood and adhering debris to the tissue; the tumor mass areas were fixed in a 10% buffered formalin solution for 7 days; then, parts of 3-5 µm were selected and fixed in paraffin and stained with hematoxylin and eosin. The evaluation was based on microscopic observations: neoplasia and dysplasia. The statistical analysis to evaluate the chemoprotective effect was the Shapiro-Wilk normality test, the non-parametric Kruskal Wallis and the Fisher tests with a 95% confidence level.

Table 4 summarizes the percentage of histopathological observations of the colon of the rats included in the study, induced with colon cancer and treated with the different suspensions. When applying the Kruskal Wallis test, with a confidence level of 95%, a p <0.05 was found in all cases, the results of the different groups show significant differences between all types of treatment and the phase of cancer development (normal, dysplasia and neoplasia). All animals treated only with DMH (100%) developed dysplasia or neoplasia, which shows the functioning of the model. The only group where the appearance of neoplasia was not observed was the one treated with the FAMC composition, and only 20% of these animals developed dysplasia, which implies that 80% of these animals were not affected by the application of the carcinogenic agent.

In the group treated with the suspensions FA, MC and RAT, only the 20, 10 and 10% of the animals, respectively, were not affected by the cancer inducing agent. In this way, it was demonstrated a relevant synergistic effect between the extract of alcohols and the MC, which gives a chemoprotective effect to the suspension, object of the present invention. Chemoprotective effect of FAMC suspension was much greater that the chemoprotective effect of the suspensions of such individual substances.

**Table 4. Histopathological results in the colon of rats treated with vehicle (PS), dimethylhydrazine (DMH) and with different suspensions (frequency percentage).**

| **Histopathological Result** | **Treatment** | **Frecuency (%)** |
|---|---|---|
| **No Changes (normal)** | Vehicle (polysorbate solution) | 100 |
| | DMH | 0 +++ |
| | DMH + suspension FAMC | 80 |
| | DMH + suspension RAT | 10 ++ |
| | DMH + suspension FA | 20++ |
| | DMH + suspension MC | 10++ |
| **Dysplasia** | Vehículo (polysorbate solution) | 0 |
| | DMH | 30+ |
| | DMH + suspension FAMC | 20 |
| | DMH + suspension RAT | 40+ |
| | DMH + suspension FA | 50++ |
| | DMH + suspension MC | 60++ |
| **Neoplasia** | Vehículo (polysorbate solution) | 0 |
| | DMH | 70+++ |
| | DMH + suspension FAMC | 0 |
| | DMH + suspension RAT | 50++ |
| | DMH + suspension FA | 30+ |
| | DMH + suspensoón MC | 30+ |

| | | |
|---|---|---|
| PS: 3% sodium polysorbate. Statistical evaluation was performed using the Kruskal-Wallis test to determine the distribution in each cancer stage group with respect to treatment categories; significance level of 0.05. Normal (p=0.01), dysplasia (p=0.049), neoplasia (p=0.01). + p<0.05; ++ p<0.01; +++ p<0.001 compared to the group with polysorbate vehicle;(Fisher test). | | |

In conclusion, the indicators of the histopathological study revealed that the suspension containing the mixture of fatty alcohols and microcrystalline cellulose (FAMC) was the only one that demonstrated significant antitumor activity in colon cancer induced by dimethylhydrazine in rats with 100% inhibition of neoplasia.

## Claims

1. Oral suspension with antiulcer and chemoprotective effect on colon cancer, **characterized by** being an aqueous suspension containing as active ingredients a purified extract of fatty alcohols from the refined wax of Apis mellifera bees and microcrystalline cellulose.

2. Oral suspension according to claim 1, **characterized in that** the ingredients are in the following proportion: 1-2% of the fatty alcohol extract; 1-2% microcrystalline cellulose; 2-4% emulsifying agents; 0.1-0.6% preserving agents; 20-30% moisturizers; 1-6% sweeteners; 0.1-1% suspending agents; 0.01-0.05% flavorings and 50-70% purified water.

3. Oral suspension according to claim 2, **characterized in that** the particle size of the purified extract of fatty alcohols from the refined beeswax is less than 1.5 micrometers.

4. Oral suspension according to claims 2 and 3, **characterized in that** the emulsifying agent is polyoxyethylene hydrogenated castor oil or polysorbate, the preserving agents are methyl and propylparabens, the wetting agent is glycerin, the sweeteners are sorbitol or sodium saccharin, the suspending agent is carboxymethylcellulose and the flavoring is an essential oil.

5. Procedure for obtaining an aqueous suspension with an antiulcer and chemoprotective effect on colon cancer, **characterized by**: A) mixing a purified extract of fatty alcohols with one or more emulsifying agents; B) subjecting said mixture to a technological process to reduce the particle size of the extract to <1.5 micrometers; C) Incorporate into the mixture with constant stirring at > 1000 rpm microcrystalline cellulose and preservative, wetting, sweetening, suspending, flavoring agents and purified water.

6. Process for obtaining an aqueous suspension, according to claim 5, **characterized in that** the fatty alcohol extract is extracted and purified from Apis mellifera beeswax.

7. Procedure for obtaining an aqueous suspension, according to claims 5 and 6, **characterized in that** the technological process applied to reduce the particle size of the fatty alcohol extract is based on subjecting the mixture of said extract and the emulsifying agent to a shearing process with a homogenizing equipment at a stirring speed > 1000 rpm, at a temperature > 80°C for > 1 minute.

8. Procedure for obtaining an aqueous suspension, according to claims 5 and 6, **characterized in that** the technological process applied to reduce the particle size of the fatty alcohol extract is based on subjecting the mixture of said extract and the emulsifying agent to a stirring speed > 1000 rpm at a temperature > 80°C for > 1 minute and then reduce the temperature to < 60°C with the same stirring speed.
